# EUROPEAN PATENT APPLICATION

(11) **EP 2 172 563 A1**
(43) Date of publication of application: **07.04.2010**
(21) Application number: 08016733.1
(22) Date of filing: 24.09.2008
(51) Int. Cl.: C12Q 1/68

(54) **Method for lowering the dependency towards sequence variation of a nucleic acid target in a diagnostic hybridization assay**

(71) Applicant: bioMérieux S.A., 69280 Marcy l'Etoile (FR)
(72) Inventor: Deiman, Birgit, 5063 EE, Oisterwijk (NL)

(57) **Abstract**

The present invention provides primer or/and probe, this target sequence containing at least one variation, defined as either one non-conserved nucleotide, called genotype variation, or one nucleotide variation within one and the same genotype, wherein the primer or/and probe comprises a nucleic acid sequence that is complementary to said target sequence except for the at least complementary base of the variation(s), which is not present in the primer or/and probe.

The present invention is especially useful in methods for diagnostic, preventive and therapeutic applications.

## Description

### FIELD OF THE INVENTION

The present invention relates to primers designed to overcome differences in amplification efficiency, especially due to primer binding variability, due to non-conserved nucleotides of the target nucleic acid sequence, for example variation between different genotypes. By deletion of the complementary base of the non-conserved nucleotide, the binding of a primer, called deletion primer, will be the same for all targets (genotypes). This invention could be transposed in other technical aspects, such as internal controls and/or detection and/or other nucleic acid sequences in competition with the target in case of quantitation assay, where amplification by the deletion primer of target, internal control and/or competitive sequences is comparable among the variants of the target nucleic acid.

### BACKGROUND OF THE INVENTION

The development of the molecular biology allows the sensitive detection of nucleic acids by using amplification technologies. The amplification methods have become very powerful tools, since they allow the exponential amplification of very small quantities of nucleic acids in a relatively short time. This generated very powerful assays with sensitivities down to single copies of RNA or DNA.

Various amplification techniques have been developed, such as PCR, LCR, NASBA, TMA and SDA, and are well known to the person skilled in the art. Each of these methods are based on different mechanisms of action, but all depend on the annealing of one or more primers to the target nucleic acid sequence or its complementary sequence.

During or after amplification of the target or part thereof, the presence or amount of amplicons generated should be detected. This can be done with various known techniques such as separation of the sample on a gel with subsequent blotting and probing. This can only be done after the amplification is finished. In a homogeneous procedure, amplification and detection occur without separating the reaction components. Amplicons are detected in the course of the amplification. Thus, the generation of amplicons can be monitored real-time and the data thus obtained can be used to determine the presence or absence and/or the amount of the amplicons.

The choice of the oligonucleotides to be used as primers and probes in the amplification and detection of nucleic acid sequences is critical for the sensitivity and specificity of the assay. The sequence to be amplified is usually only present in a sample (for example a blood sample obtained from a patient suspected of having a viral infection) in minute amounts. The primers should be sufficiently complementary to the target sequence to allow efficient amplification of the viral nucleic acid present in the sample. If the primers do not anneal properly (due to mispairing of the bases on the nucleotides in both strands) to the target sequence, amplification is seriously hampered. When the target is not linear but has a particular structure, it is less accessible by the primer, which in turn also leads to a reduced amplification. This will affect the sensitivity of the assay and may result in false negative test results.

Efficient hybridization of the primer generally requires at least 15 contiguous complementary nucleotides. If less nucleotides are present, the ratio between specific and non-specific annealing of the primer decreases below a point where no longer effective amplification of the target nucleic acid can occur. If several genetic variations of a target nucleotide sequence are known, conserved stretches of nucleotides can be identified and used for primer design. However, preferred conserved stretches in some species, like RNA viruses, such as parts of the coding region of the human hepatitis C virus, are sometimes too small to be used to design efficient primers. In addition, even these conserved segments have a tendency to mutate and unknown polymorphisms are continuously generated. This is most pronounced in fast dividing organisms like viruses and bacteria, resulting in a reduced efficiency of hybridization of the primer. As a consequence, amplification may not occur, which is highly undesirable in for example routine diagnostics since a false negative amplification result can have severe consequences for the patient concerned. Also, in case of quantitative assays, sequence variations (polymorphisms) in the target can lead to under-quantifications thereof.

In addition, sequence differences between the amplified target sequence and the probe used for detection further lower the efficiency of detection. Targets with polymorphisms, are thus less well detected than targets that match perfectly with the consensus sequence of the probe.

The document EP-A-1.426.448 refers to a method for lowering the effect of sequences variations in diagnostic hybridization assays that use a nucleic acid probe to detect an amplified nucleic acid analyte. It consists in introduction of one or more nucleotides that have an affinity increasing modification, and more particularly in the loop of the molecular beacon. The sensitivity to polymorphisms is lowered because the affinity of the probe to the polymorphic analyte is increased.

Nevertheless, this method is dedicated to the probes and does not overcome the mismatching of the primer to the target containing at least one nucleotide variation. Furthermore, regarding the design of the probes the solution proposed requires modified nucleotides, which are expensive and needs a specific manufacturing process.

The document EP-B-0.246.864 relates to a method for discriminating between alternative nucleotide sequences wherein two nucleotide probes are used and form a split probe hybrid with a complementary target sequence. The two probes being such that a potential mismatch in the target sequence lies between said probes.
As the document mentioned above, one drawback of this method is that it is dedicated only to probes and not to primers. Furthermore, this method requires the use and the design of two probes.

Therefore there is a clear need for an amplification method, which is less prone to mutations or genetic variations in the hybridizing segment of the target sequence.

### SUMMARY OF THE INVENTION

The present invention provides primers and/or probes, which are a very versatile tool for lowering the dependency towards sequence variation of the target in a homogenous assay. According to a first embodiment, the present invention provides primer designed to amplify target nucleic acid sequence, this target sequence containing at least one variation, defined as either one non-conserved nucleotide, called genotype variation, or one nucleotide variation within one and the same genotype, wherein the primer comprises a nucleic acid sequence that is complementary to said target sequence, except for the at least complementary base of the variation(s), which is not present in the primer.

In a particular embodiment, the primer is complementary of the target that contains at least two variations next to each other.

According to one embodiment, the primer is complementary to the target sequence that contains at least two variations, each variation is separated from each other variation by at least one conserved nucleotide.

According to another embodiment, this primer can form with another one a primer pair. Thus, the present invention provides a primer pair to amplify target nucleic acid sequence, this target sequence containing at least one variation, defined as either one non-conserved nucleotide, called genotype variation, or one nucleotide variation within one and the same genotype, wherein at least one of the primers is designed according to the present invention. Thus, the amplification is performed by contacting the sample with a pair of primers, called the forward and the reverse primers respectively P1 and P2 in NASBA approach. When NASBA process is performed, the P1 primer will hybridize downstream of the sequence of interest, whereas the P2 primer will be complementary to the strand generated by the elongation of the forward primer and will be positioned upstream of said sequence of interest. In case of PCR amplification is performed, the forward primer being upstream and the reverse primer being downstream of the sequence of interest.

Another object of the invention is a probe designed to detect target nucleic acid sequence, this target sequence containing at least one variation, defined as either one non-conserved nucleotide, called genotype variation, or one nucleotide variation within one and the same genotype, wherein the probe comprises a nucleic acid sequence that is complementary to said target sequence except for the at least complementary base of the variation(s), which is not present in the probe.

According to one embodiment, the probe comprises at least one label. Various labeling moieties are known in the art. Said moiety may, for example, either be a radioactive compound, a detectable enzyme (e.g. horse radish peroxidase (HRP)), a hapten like biotin, or any other moiety capable of generating a detectable signal, such as a colorimetric, fluorescent, chemiluminescent or electrochemiluminescent signal.

According to any embodiments of the primer or probe, the target sequence is a RNA target.

Again, according to another embodiment, primer is provided with a promoter sequence. The term "promoter sequence" defines a region of a nucleic acid sequence that is specifically recognized by an RNA polymerase that binds to a recognized sequence and initiates the process of transcription by which an RNA transcript is produced. In principle any promoter sequence may be employed for which there is a known and available polymerase that is capable of recognizing the initiation sequence. Known and useful promoters are those that are recognized by certain bacteriophage RNA polymerases, such as bacteriophage T3, T7 or SP6. Oligonucleotides linked to a promoter sequence are commonly referred to as "promoter primers". Their function as a primer, e.g. the starting point for an elongation reaction, however, may be blocked, as already mentioned above, or absent in some embodiments of transcription based amplification reactions.

The present invention further provides a method of amplifying in a sample a nucleic acid target sequence containing at least one variation, defined as either one non-conserved nucleotide, called genotype variation, or one nucleotide variation within one and the same genotype, comprising performing on the sample an amplification reaction that amplifies the presence of this target sequence utilizing
1) a first primer capable of specifically hybridizing in a region of the target sequence and capable of directing, under extension conditions, extension of the primer toward the hybridizing region of a second primer, and
2) the second primer capable of specifically hybridizing to a complementary sequence of another region of the target sequence and capable of directing, under extension conditions, extension of the second primer toward the complementary sequence of the hybridizing region of said first primer, and
wherein said at least one variation is located in the hybridizing region of at least one of the primers, and
wherein at least one of the primers comprises a nucleic acid sequence that is complementary to said target sequence except for the at least complementary base of the variation(s), which is not present.

According to another embodiment, the method of the invention may be of any kind in which primers and probe are used to detect a nucleic acid analyte. Such assays may be based on the detection of amplified analytes, such as in PCR-, TMA- or NASBA-based assays. However, the primers and probe can also be used in arrays. The invention can be used both in quantitative and qualitative diagnostic assays in which sequence polymorphisms of the target influence the reliability of the assay.

Another object of the invention is a method of detecting in a sample a nucleic acid target sequence containing at least one variation, defined as either one non-conserved nucleotide, called genotype variation, or one nucleotide variation within one and the same genotype, comprising
(a) performing on the sample an amplification reaction that detects the presence of this target sequence utilizing
   1) a first primer capable of specifically hybridizing in a region of the target sequence or to a complementary region of the target sequence and capable of directing, under extension conditions, extension of the first primer toward the hybridizing region of a second primer, and
   2) the second primer capable of specifically hybridizing in another region of the target sequence or to another complementary region of the target sequence and capable of directing, under extension conditions, extension of the second primer toward the complementary sequence of the hybridizing region of said first primer, and
(b) performing on the sample a detection reaction that detects the presence of said target sequence, with at least one probe capable of specifically hybridizing to a region of the target sequence between the region with which the first primer is capable of hybridizing and the region with which the second primer is capable of hybridizing,
wherein said at least one variation is located in the hybridizing region of at least one of the primers, and
wherein at least one of the primers comprises a nucleic acid sequence that is complementary to said target sequence except for the at least complementary base of the variation(s), which is not present.

According to another embodiment of the method, the amplification reaction is selected from the group consisting of transcription-based amplification and PCR.

In a particular embodiment of the method, the transcription-based amplification reaction is a NASBA reaction. The NASBA process is described in European patent no. EP-B-0.329.822. Although this process allows amplification of RNA it can be used to amplify double stranded nucleic acid as described in the European patent application EP-A-1.366.179. A major advantage of these methods over PCR is that they are performed isothermally.

According to another embodiment of the method, the target sequence is a RNA target.

Another object of the invention is a method of detecting in a sample a nucleic acid target sequence containing at least one variation, defined as either one non-conserved nucleotide, called genotype variation, or one nucleotide variation within one and the same genotype, comprising
(a) at least one probe capable of specifically hybridizing to a region of the target sequence, and
(b) detects the presence of said target sequence,
wherein said at least one variation is located in the hybridizing region of at least one of the probe(s), and
wherein at least one of the probes comprises a nucleic acid sequence that is complementary to said target sequence except for the at least complementary base of the variation(s), which is not present.

According to the last embodiment of the method, an amplification reaction is performed prior to the detection reaction that detects the presence of the amplicons issued from the target sequence.

According to another embodiment of the method, the amplification reaction consists in
(a) adding a first primer capable of specifically hybridizing in a region of the target sequence or to a complementary region of the target sequence and capable of directing, under extension conditions, extension of the first primer toward the complementary region of the target sequence where a second primer can hybridize, called the hybridizing region of a second primer, and
(b) adding, possibly simultaneously to the addition of the first primer, the second primer capable of specifically hybridizing in another region of the target sequence or to another complementary region of the target sequence and capable of directing, under extension conditions, extension of the second primer toward the complementary region of the target sequence where the first primer can hybridize, called the complementary of the hybridizing region of the first primer, and
(c) submitting the sample to reagents, such as enzymes, nucleotides triphosphates, and to physical conditions, such as temperature, pH, in order to permit hybridization and elongation of said primers and release of the amplicons thus obtained.

According to another embodiment of the method the hybridizing region of the probe is located between the region with which the first primer is capable of hybridizing and the region with which the second primer is capable of hybridizing onto to the target sequence or on to the complementary sequence of said target sequence.

According to another embodiment of the method the probe contains at least one label.

According to another embodiment of the method the probe is a nucleic acid bearing one label at each of its ends. The probe according to the invention can be a so-called molecular beacon (MB). These probes recognize their targets with higher specificity than linear probes. A class of oligonucleotide probes, referred to as molecular beacons, that facilitate homogeneous detection of specific nucleic acid target sequences has been described (Piatek et al. (1998) Nature Biotechnology 16:359-363; Tyagi and Kramer (1996) Nature Biotechnology 14:303-308). Molecular beacons are single-stranded oligonucleotides having a stem-loop structure. The loop portion contains the sequence complementary to the amplicon (either DNA or RNA). The stem is formed due to hybridisation of the complementary sequence of the 3'-end with the 5'-end. The stem can be unrelated to the amplicon and is double-stranded. One arm of the stem is labelled with a fluorescent dye (fluorophore) at its freed end, whereas the other one is coupled to a quenching moiety also at its freed end. In the stem-loop state the probe produces only little fluorescence because the energy of the fluorophore is transferred to the quenching molecule. When the molecular beacon hybridises to the amplicon the stem-loop structure is lost and the quencher and fluorophore become separated. At that stage the fluorescence emitted by the fluorophore is no longer quenched and can be detected and quantified.

According to another embodiment of the method detection reaction is a real-time detection.

Additionally, the present invention provides a kit containing at least one primer pair and at least one probe as above described.

### Definitions

According to the present invention, the term "nucleic acid" (NA) means both DNA and RNA, both in any possible configuration, i.e. in the form of double-stranded (ds) nucleic acid, or in the form of single-stranded (ss) nucleic acid, or as a combination thereof. Such nucleic acid corresponds to a succession of at least two deoxyribonucleotides or ribonucleotides optionally comprising at least one modified nucleotide.

This polynucleotide may also be modified at the level of the internucleotide bond, such as, for example, phosphorothioates, H-phosphonates, alkyl phosphonates, at the level of the backbone such as, for example, alpha-oligonucleotides (FR-A-2 607 507) or PNAs (M. Egholm et al., J. Am. Chem. Soc., 114, 1895-1897, 1992) or 2'-O-alkylriboses. Each of these modifications may be taken in combination as long as at least one phosphate is present in the nucleic acid.

The nucleic acid may be natural or synthetic, an oligonucleotide, a polynucleotide, a nucleic acid fragment, a ribosomal RNA, a messenger RNA, a transfer RNA, a nucleic acid obtained by an enzymatic amplification technique such as:
- PCR (Polymerase Chain Reaction), described in patents U.S. Pat. No. 4,683,195, U.S. Pat. No. 4,683,202 and U.S. Pat. No. 4,800,159, and its RT-PCR (Reverse Transcription PCR) derivative, in particular in a one-step format as described in patent EP-B-0,569,272,
- RCR (Repair Chain Reaction), described in patent application WO-A-90/01069,
- 3SR (Self Sustained Sequence Replication) with patent application WO-A-90/06995,
- NASBA (Nucleic Acid Sequence-Based Amplification) with patent application WO-A-91/02818, and
- TMA (Transcription Mediated Amplification) with U.S. patent No 5,399,491.

Such "nucleic acid" may be used as target, primer or probe.

The "target sequence" is defined as the part of the nucleic acid molecule to be detected. It is amplified by means of the primers and the amplification related enzymes. The amplification leads to formation of "amplicons", which are the nucleic acid molecules that are physically detected by hybridisation to the probe. Its length is up to 30 nucleotides and preferably between 100 and 300 nucleotides but longer polynucleotides, up to 1500 nucleotides, can be considered.

The term "primer" as used herein refers to an oligonucleotide either naturally occurring (e.g. as a restriction fragment) or produced synthetically, which is capable of acting as a point of initiation of synthesis of a primer extension product, which is complementary to a part of a nucleic acid strand (template or target sequence) when placed under suitable conditions (e.g. buffer, salt, temperature and pH) in the presence of nucleotides and an agent for nucleic acid polymerization, such as an enzyme, for instance DNA dependent or RNA dependent polymerase. Normally a set of primers will consist of at least two primers, one "upstream" primer and one "downstream" primer, which together define the amplicon (the sequence that will be amplified using said primers and that is a part of or complementary to a part of the target sequence). One of the primers hybridises to the (+) strand, while the second one hybridises to the (-) strand of the target. The length of the primer should be between 10 and 50, preferably between 15 and 30 nucleotides. A primer could additionally be associated to a promoter sequence for instance a T7 promoter.

As used herein the term "probe" is intended to comprise a stretch of nucleotides hybridising to the amplicon. Preferably the hybridising part is a stretch of 10-50, more preferably 15-35, most preferably 15-30 nucleotides.

In this application the terms "analyte", "amplicon" and "target" or "target sequence" may be used interchangeably. The analyte is the original nucleic acid molecule to be detected. The target sequence is the part of the analyte that is amplified by means of the primers. The amplification leads to formation of amplicons, which are the nucleic acid molecules that are physically detected by hybridisation to the probe. The sequence of the amplicons is the same or complementary to the target sequence within the analyte.

The term "non-conserved nucleotide" or "genotype variation" or "one nucleotide variation" or "variation" refers to any single or multiple nucleotide substitutions, deletions or insertions. These nucleotide variations may be mutant or polymorphic allele variations.

As used herein, the term "polymorphism" refers to the condition in which two or more different nucleotide sequences can exist at a particular site in DNA or RNA.

The present invention will be further illustrated in the Examples that follow and which are not intended to limit the invention in any way.

### BRIEF DESCRIPTION OF THE DRAWINGS

**Figure 1** shows HCV NASBA amplification with a deletion p1 primer: a comparison of two graphs corresponding to the amplification by a normal p1a/p2b primer pair (Figure 1A) and by a p1d/p2b primer pair (Figure 1B) according to the invention, wherein the p1d contains one deletion.
**Figure 2** demonstrates HCV NASBA amplification with various deletion p1 primers: a comparison of four graphs corresponding to the amplification by a normal p2 primer (HCV-E2 p2b) associated in a primer pair with a p1, which is:
   - a deletion primer HCV-E2-pld containing one deletion, as exposed in Figure 2A,
   - a deletion primer HCV-E2-p1e containing two deletions, as exposed in Figure 2B,
   - a deletion primer HCV-E2-p1f containing two deletions, as exposed in Figure 2C, and
   - a deletion primer HCV-E2-p1g containing three deletions as exposed in Figure 2D.

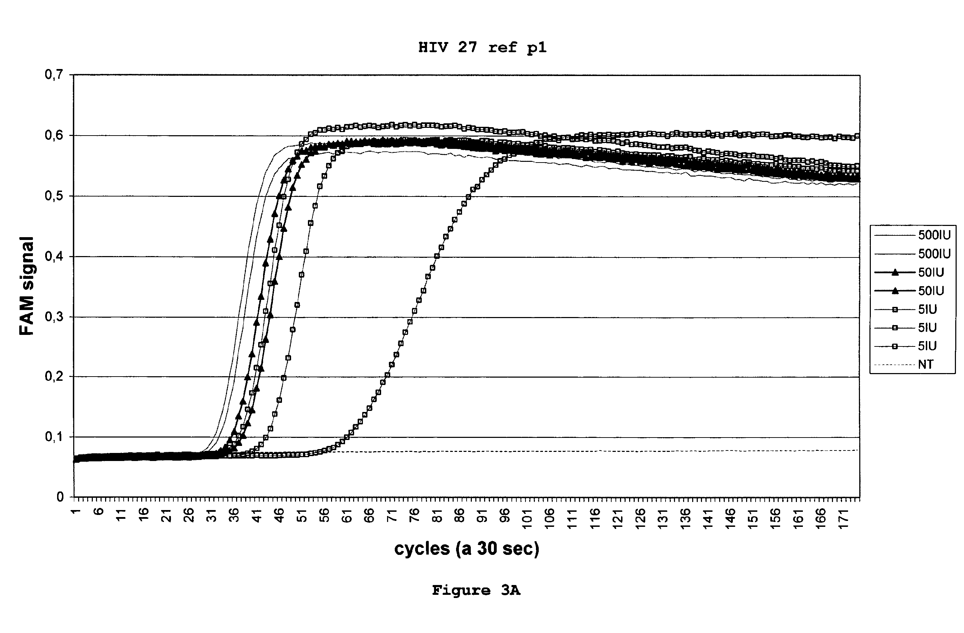

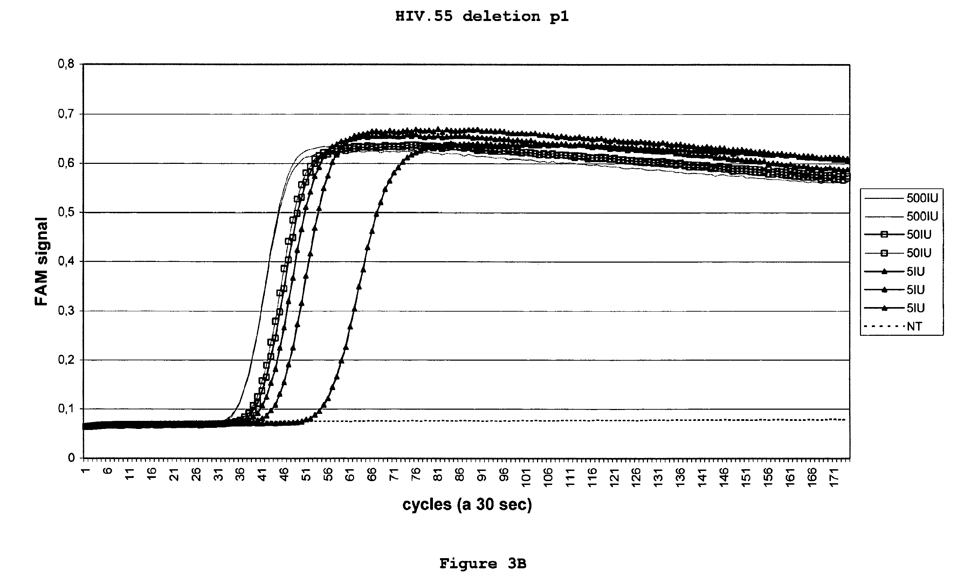

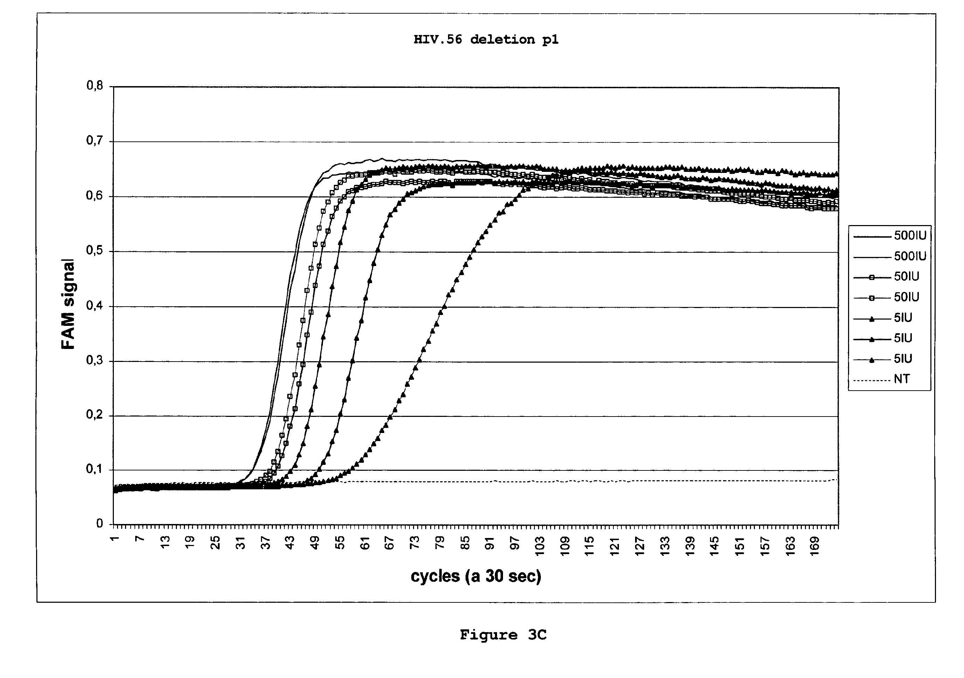

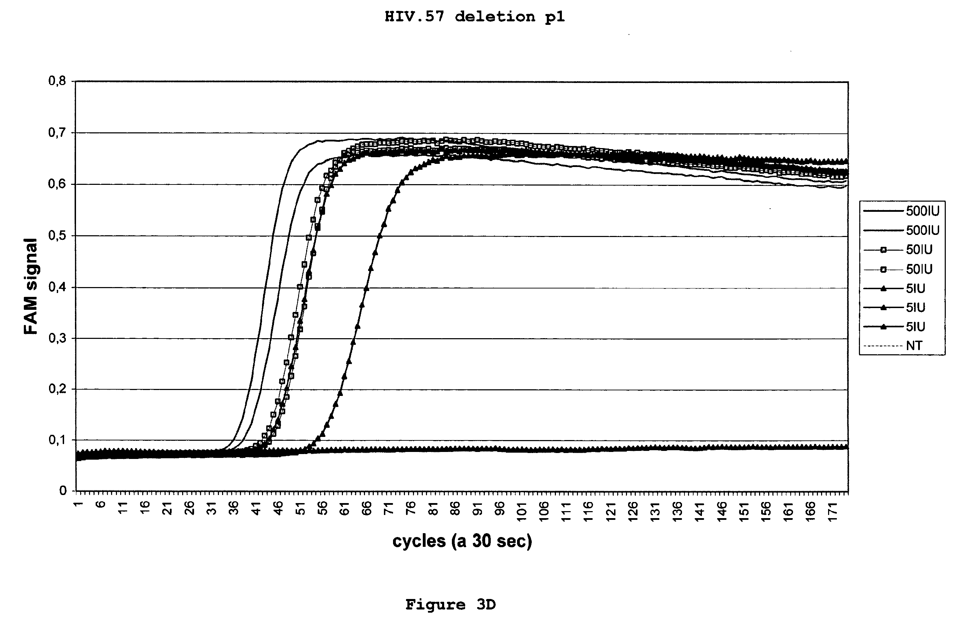

**Figure 3** exposes amplification of HIV RNA with various deletion p1 primers: a comparison of four graphs corresponding to the amplification by a normal p2 primer (HIV-E2 p2b) associated in a primer pair with a p1, which is:
   - a normal primer HIV.27 containing no deletion, as exposed in Figure 3A,
   - a deletion primer HIV.55 containing one deletion, as exposed in Figure 3B,
   - a deletion primer HIV.56 containing one deletion, as exposed in Figure 3C, and
   - a deletion primer HIV.57 containing two deletions as exposed in Figure 3D.

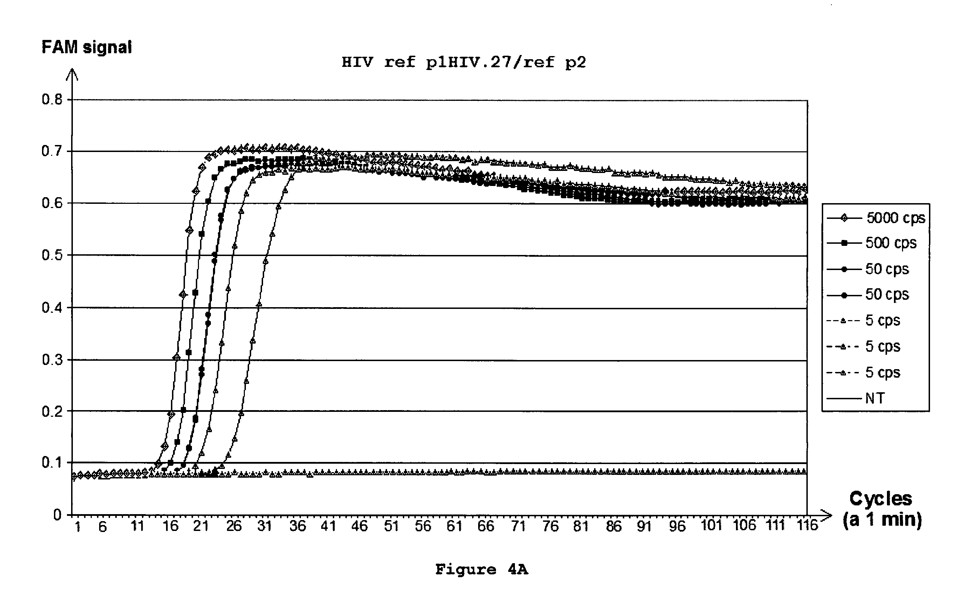

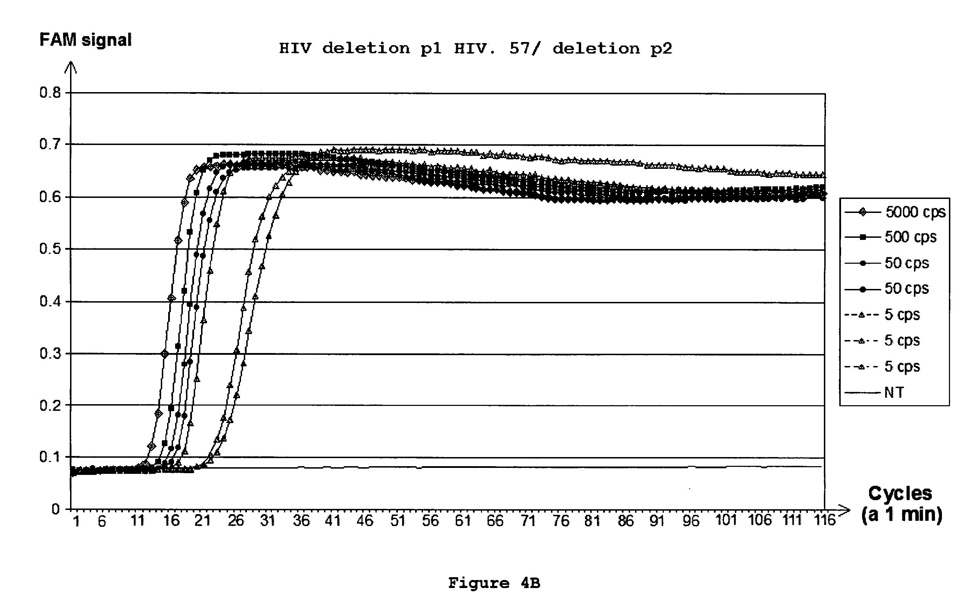

The two charts associated with **figures 4** show NASBA amplification of HIV RNA with deletion p1 and p2 primers.

HIV amplification was performed with a deletion primer set, HIV deletion p1 HIV.57 and HIV deletion p2 (table 1) both including two deletions (fig. 4b). The reference primer set used is HIV ref p1 HIV.27 in combination with HIV ref p2 (table 1) both without deletions (fig. 4a). Amplification was performed in presence of a HIV specific molecular beacon (HIV WT MB, Me23). 5000, 500, 50 and 5 copies of in vitro transcribed HIV subtype B RNA was used as input material for amplification. A sample without template (NT) was used as negative control.

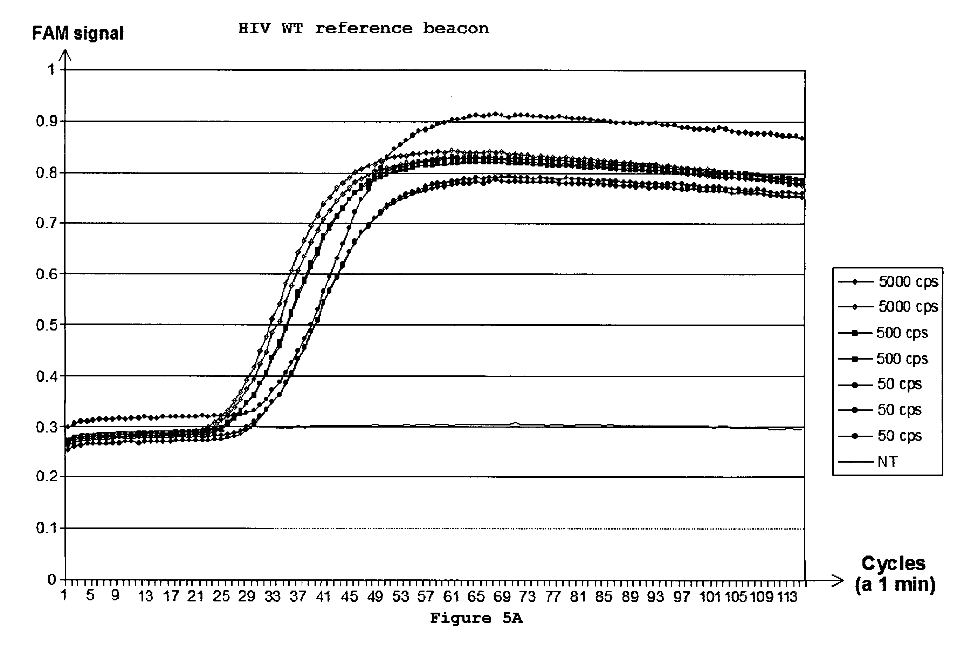

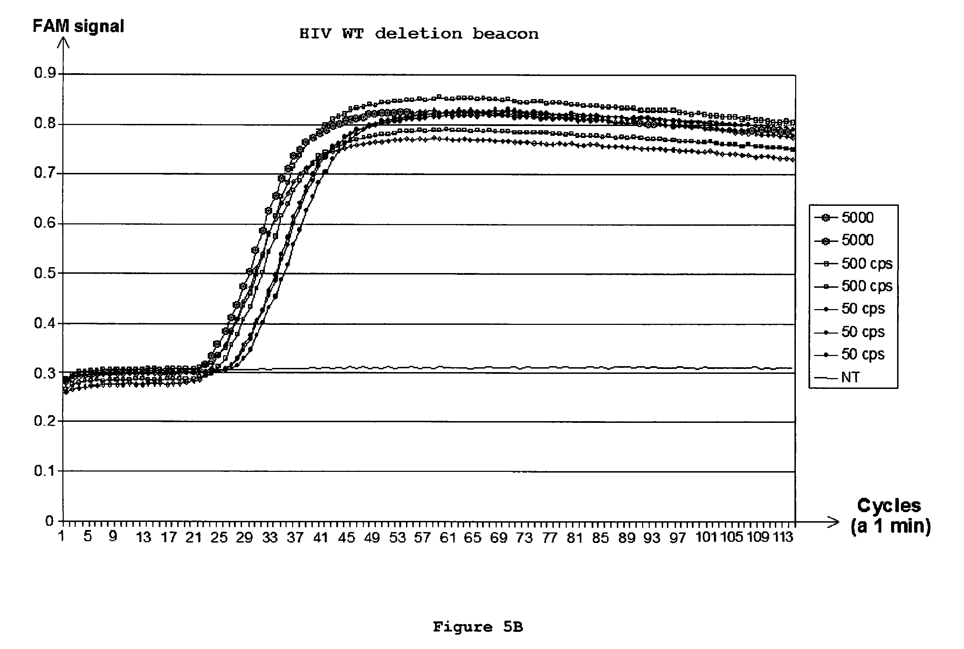

The two charts associated with **Figures 5** demonstrate NASBA amplification of HIV RNA in presence of a deletion molecular beacon. HIV amplification with a reference primer set (HIV ref p1 HIV.27 and HIV ref p2) was performed in presence of a HIV specific molecular beacon (HIV WT MB, OTMB8472, table 1) indicated as "HIV WT reference beacon" (fig. 5a) or in presence of a HIV deletion beacon including two deletions (HIV WT deletion MB, table 1) indicated as "HIV WT deletion beacon" (fig. 5b). 5000, 500 and 50 copies of in vitro transcribed HIV subtype B RNA was used as input material for amplification. A sample without template (NT) was used as negative control.

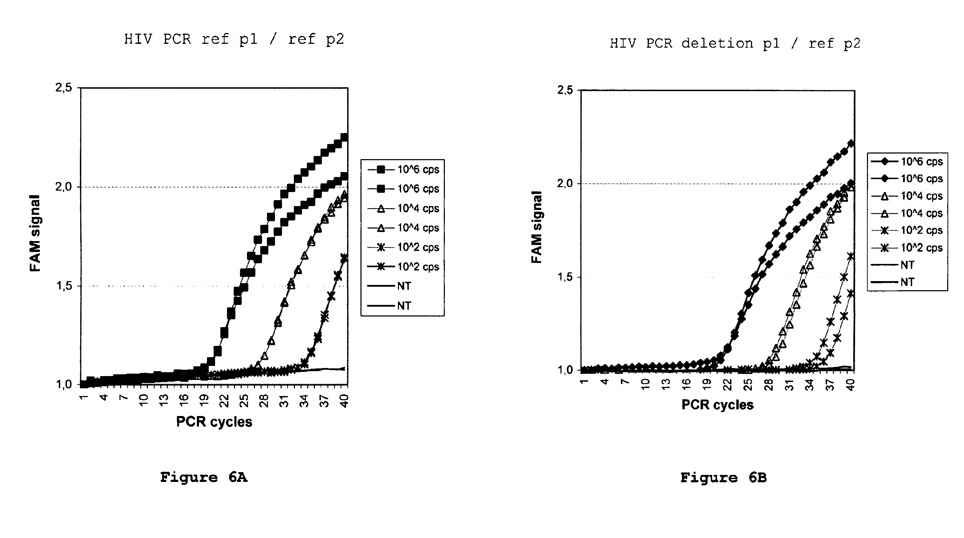

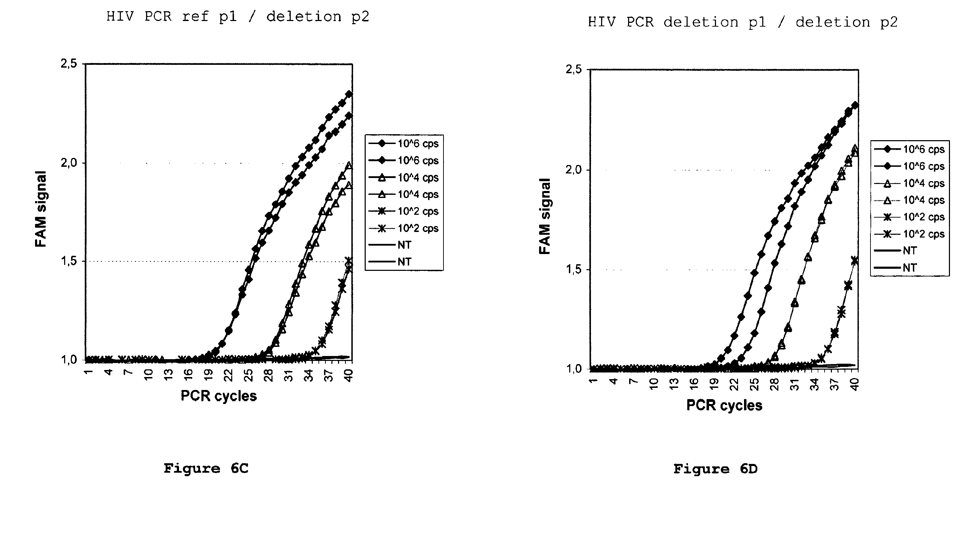

**Figures 6** expose PCR amplification of HIV DNA with deletion primers. HIV PCR amplification was performed using various primer combinations, with and without deletions, in presence of a HIV specific molecular beacon (HIV WT MB (MB41, table 1)). Two deletion primers were used, HIV PCR deletion p1 and HIV deletion p2, both including two deletions. Four different primer combinations were tested:
Fig. 6a: Reference primer set, HIV PCR ref p1 with HIV ref p2,
Fig. 6b: HIV PCR deletion p1 with HIV ref p2,
Fig. 6c: HIV PCR ref p1 with HIV deletion p2 and
Fig. 6d: HIV PCR deletion p1 with HIV deletion p2. A dilution series of approximately 10⁶, 10⁴ and 100 copies of plasmid DNA containing partly HIV subtype B sequence, was used as input material for amplification. A sample without template (NT) was used as negative control.

The sequences of the p1 primers, p2 primers and Molecular beacons, used in the examples 1-6 presented below are for NASBA amplification and are summarized in Table 1.

| | ***Sequence 5'-> 3'*** |
|---|---|
| *HCV p1 primers* | |
| HCV-E2 pla: seq ID NO I | *AATTCTAATACGACTCACTATAGGG* AGA **GTTCATCCACGTGCAGCCGAACCA** |
| HCV-E2 pld: seq ID NO 2 | *AATTCTAATACGACTCACTATAGGG* AGA **GTTCATCCACGTGCA*CCGAACCA** |
| **HCV-E2 p1e:** seq ID NO 3 | *AATTCTAATACGACTCACTATAGGG* AGA **GTTCATCCACG*GCA*CCGAACCA** |
| **HCV-E2 p1f:** seq ID NO 4 | *AATTCTAATACGACTCACTATAGGG* AGA **GTTCATCCA*GTGCA*CCGAACCA** |
| **HCV-E2 p1g:** seq ID NO 5 | *AATTCTAATACGACTCACTATAGGG* AGA **GTTCATCCA*G*GCA*CCGAACCA** |

| *HCV p2 primer* | |
|---|---|
| **HCV-E2 p2b:** seq ID NO 6 | GGCCGTATTGTTGGCATTAT |

| *HCV WT molecular beacon* | |
|---|---|
| **HCV-E2 ref MB:** seq ID NO 7 | *5' FAM* cgac**TG TGGCCCGGTATATTGCTT Ca**gtcg-*3' dabSyl* |

| *HIV p1 primers* | |
|---|---|
| **HIV.27 HIV ref p1:** seq ID NO 8 | *AATTCTAATACGACTCACTATAGGG* AGA CCTGCTATGTCACTTCCCCTTGGTTCTCT |
| **HIV.55 HIV deletion p1:** seq ID NO 9 | *AATTCTAATACGACTCACTATAGGG AGA* **CCTGCTAT*TCACTTCCCCTTGGTTCTCT** |
| **HIV.56 HIV deletion p1:** seq ID NO 10 | *AATTCTAATACGACTCACTATAGGG AGA* **CCTGCTATGTCACTTCCCCT*GGTTCTCT** |
| **HIV.57 HIV deletion p1:** seq ID NO 11 | *AATTCTAATACGACTCACTATAGGG AGA* **CCTGCTAT*TCACTTCCCCT*GGTTCTCT** |

| *PCR primers p1-T7* | |
|---|---|
| **HIV PCR ref p1:** seq ID NO 12 | CCTGCTATGTCACTTCCCCTTGGTTCTCT |
| **HIV PCR deletion p1:** seq ID NO 13 | **CCTGCTAT*TCACTTCCCCT*GGTTCTCT** |

| *HIV p2 primer* | |
|---|---|
| **HIV ref p2:** seq ID NO 14 | **AGTGGGGGGACATCAAGCAGCCATGCAAA** |
| **HIV deletion p2 (p2.20) :** seq ID NO 15 | **AGTGGGGGGACATCA*GCAGC*ATGCAAA** |

| *HIV WT molecular beacon* | |
|---|---|
| **HIV WT MB (Me23) :** seq | *5' FAM*-ctatccc **ATCAATGAGGAIGCTGCAGAIT**-gggatag*3' dabSyl* ID NO 16 |
| **HIV WT MB (Me41) :** seq | *5'FAM* ctatccc **ATCAATGAGGAIGCTGCAGAAT** gggatag *3'dabSyl* ID NO 17 |
| HIV WT MB **(OTMB8472):** seq ID NO 18 | *5'FAM t*gcatgc ATCAATGAGGAIGCTGCAGAITGGGA gcatgc *3'dabSyl* |
| **HIV WT deletion MB:** seq ID NO 19 | *5'FAM-*cgatgc **ATCAATGAGGA*GCTGCAGA*TGGGA** gcatcg *3'dabSyl* |

Target specific sequences are presented in bold, T7 promoter sequences are in italic, 2'-O-methoxy nucleotides are underlined and finally the nucleotides present between target specific sequences and T7 promoter sequences, which are neither in bolt nor in italic, are linkers. Finally the stars "*" represent the position where one nucleotide has been deleted.

### Example 1: Amplification of HCV RNA with a deletion p1 primer

Part of the E2 region of HCV was amplified using two different p1 primers, HCV-E2 p1a or HCV-E2 p1d (table 1), in combination with p2 primer HCV-E2 p2b (table 1) and in presence of a HCV specific molecular beacon HCV-E2 ref MB (table 1). Primer HCV-E2 p1d is similar to primer p1a except for a one-nucleotide deletion at the position where nucleotide variation occurs between different HCV subtypes. This primer HCV-E2 p1d is nominated as "deletion primer".

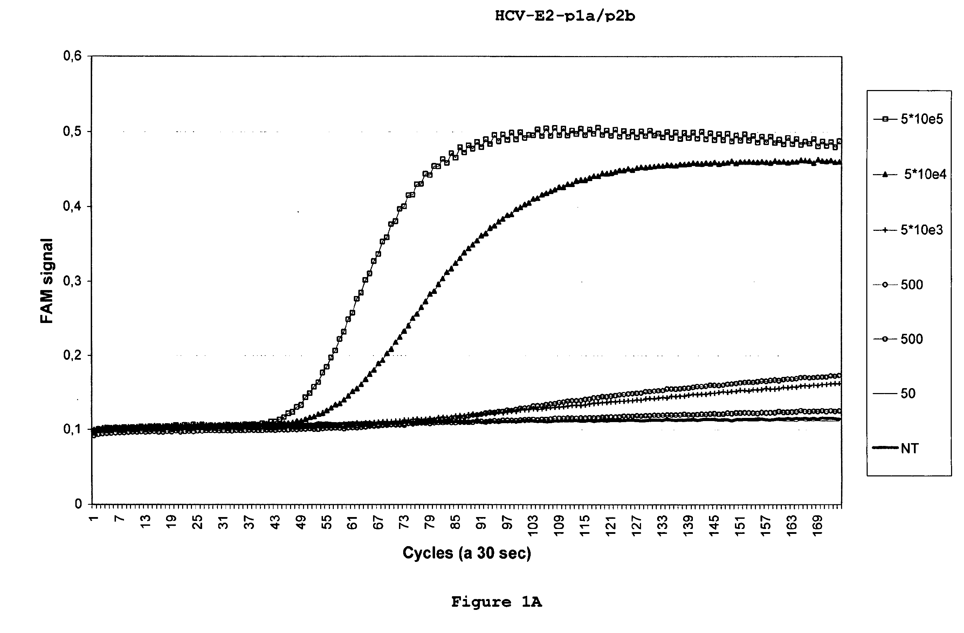

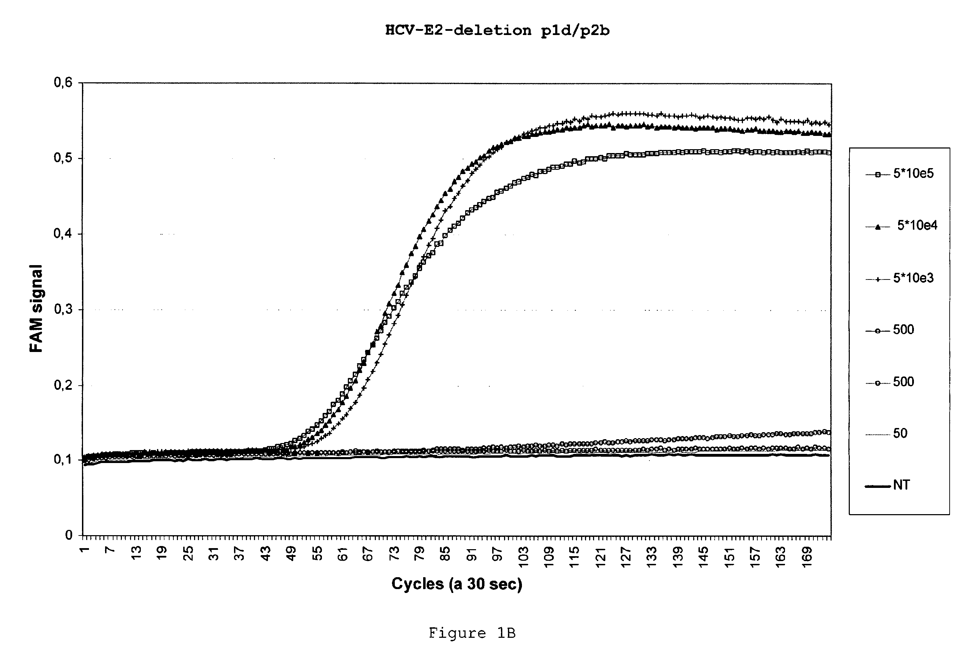

A dilution series, 5.10⁵ to 50 copies, of *in vitro* transcribed HCV RNA was used as input material for amplification. Amplification was performed using standard EasyQ Basic Kit NASBA reagents (bioMérieux B.V., Boxtel, The Netherlands) (40 mM Tris-HCl pH 8.5, 12 mM MgCl₂, 80 mM KCl, 15% v/v DMSO, 5 mM DTT, 1 mM each dNTP, 2 mM ATP, 2 mM CTP, 2 mM UTP, 1.5 mM GTP, 0.5 mM ITP, 0.2 µM of each primer and 0.01 µM of the molecular beacon probe). The mixture was incubated for 2 min at 65°C to denature the RNA and for 2 min at 41°C, to hybridize the P1 primer to the target. Subsequently, NASBA enzymes were added, the reaction mixture was mixed by gently vortexing and short centrifugation, and the amplification and real-time detection was started. The reaction mixture was incubated at 41°C in the NucliSens EasyQ Analyzer (NucliSens, BioMérieux) for 90 minutes with fluorescence monitoring every 30 seconds. The reactions were excited at 485 nm and the emission signal was measured at 518 nm. Results are presented in figure 1. The results show that amplification with a p1 deletion primer HCV-E2-pld, as exposed in Figure 1B, is possible and even a better assay sensitivity was obtained with the deletion p1 primer as compared to the reference p1 primer HCV-E2-p1a, as presented in Figure 1A.

### Example 2: Amplification of HCV RNA with various deletion p1 primers

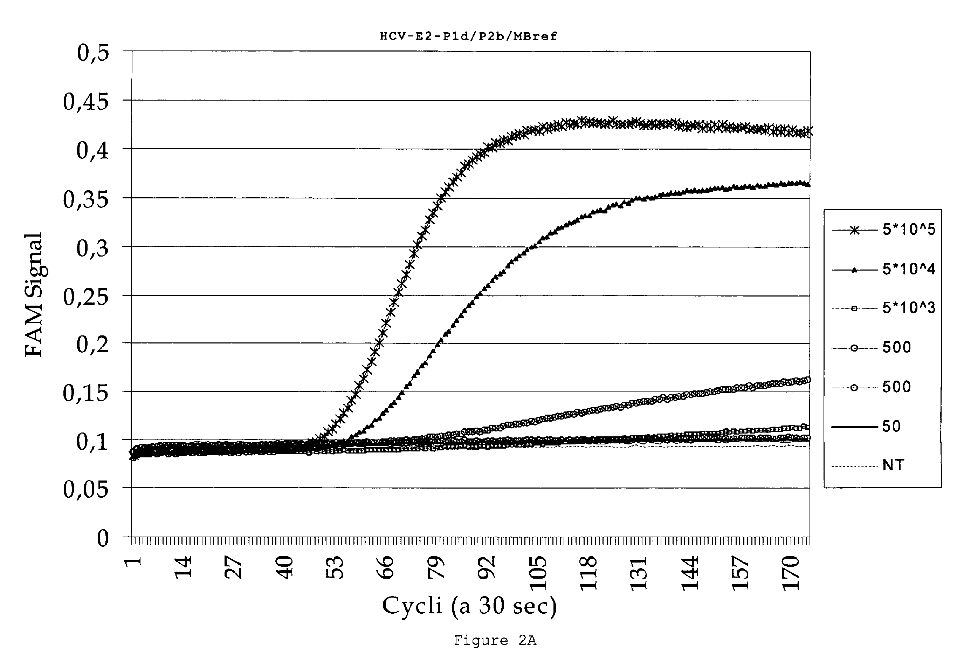

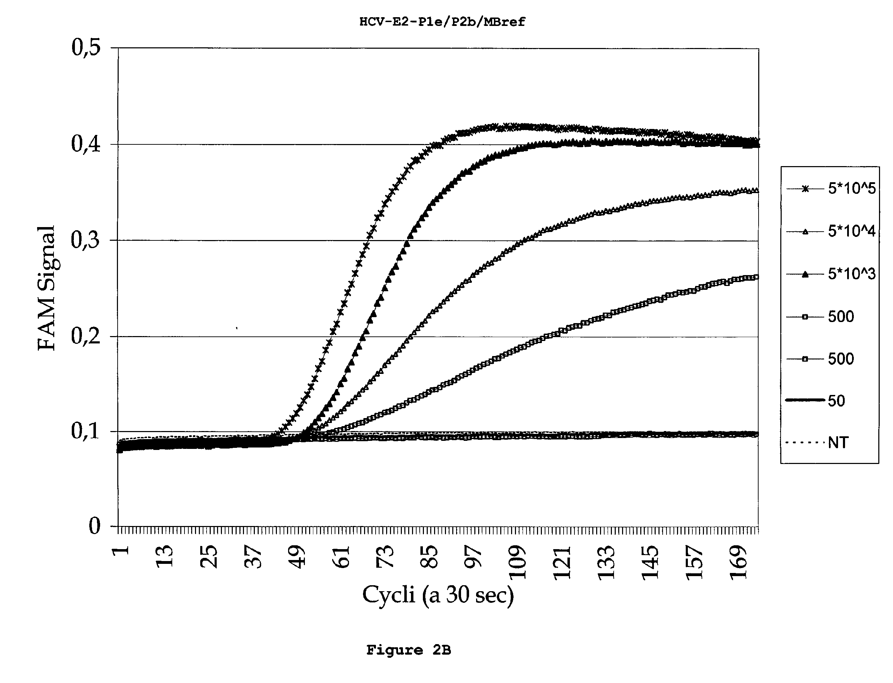

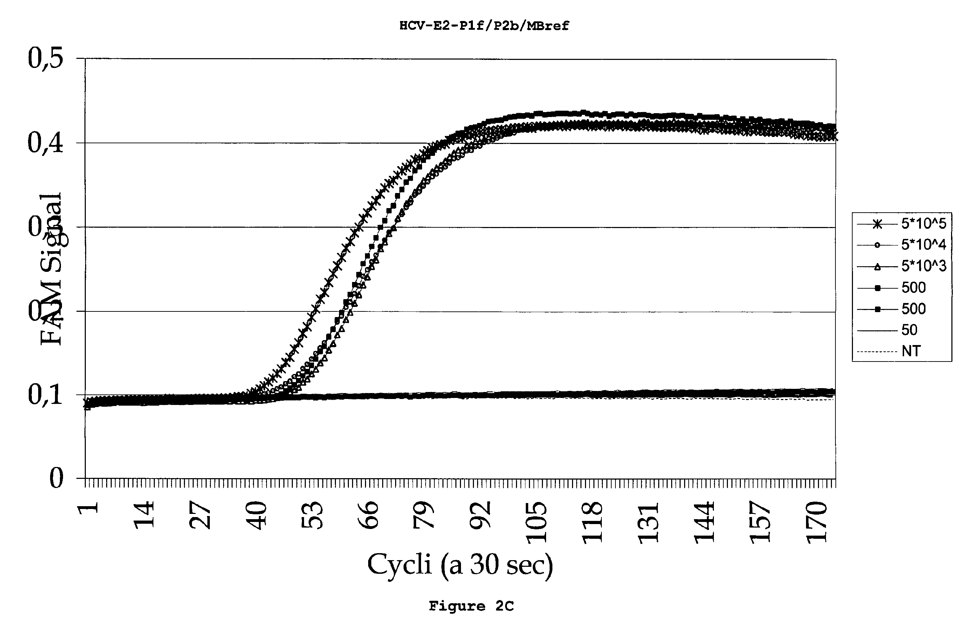

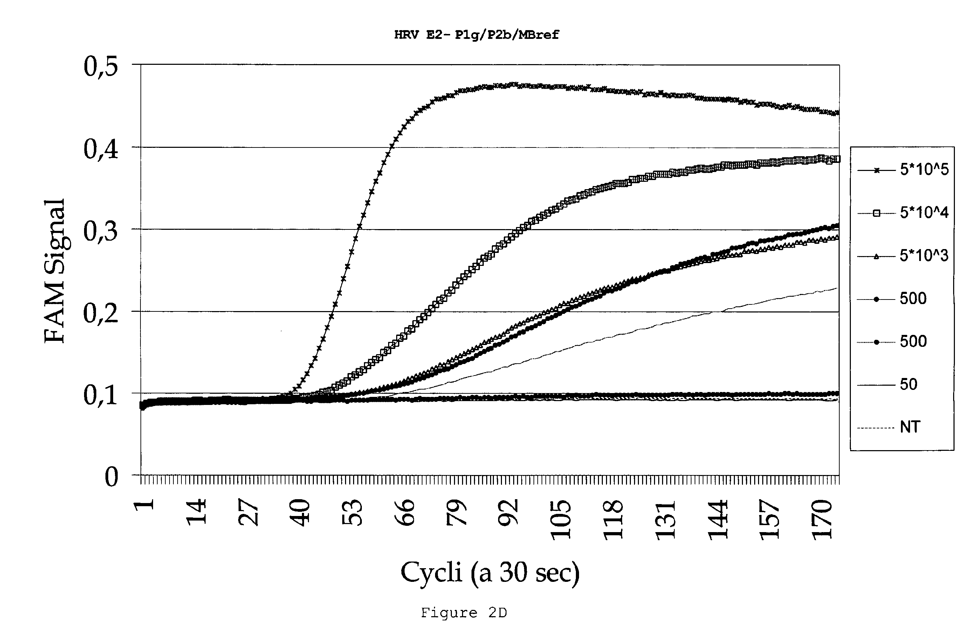

HCV amplification was performed using various HCV-E2 p1 deletion primers in combination with p2 primer HCV-E2 p2b (table 1) and in presence of a HCV specific molecular beacon HCV-E2 ref MB (table 1). Besides HCV-E2-pld, new deletion primers HCV-E2 p1e, HCV-E2 p1f and HCV-E2 p1g were tested. HCV-E2 p1d includes one deletion, HCV-E2 p1e and p1f two deletions and HCV-E2 p1g three deletions, all at positions where nucleotide variation occurs between different HCV subtypes (table 1). A dilution series, 5.10⁵ to 50 copies, of *in vitro* transcribed HCV RNA was used as input material for amplification. Amplification was performed as described in example 1. Results are presented in figure 2.

The results show that amplification with p1 primers including up to three deletions is possible with equal or even better assay sensitivity as compared to primer HCV-E2 p1d primer. HCV-E2 p1f (Figure 2C), including two deletions, shows the best assay kinetics.

### Example 3: Amplification of HIV RNA with various deletion p1 primers

Amplification with various HIV deletion p1 primers was performed in combination with p2 primer HIV-ref p2 (table 1) and in presence of a HIV specific molecular beacon (HIV WT MB(Me23), table 1). The p1 primers used are HIV.55 and HIV.56 (table 1) including a one-nucleotide deletion and HIV.57 (table 1) including two deletions at positions where nucleotide variation occurs between different HIV subtypes. P1 primer HIV.27 without deletions was used as reference. HIV particles, from cell culture, lysed with lysis buffer (NucliSens Extraction reagents, bioMérieux, Boxtel, The Netherlands) were used as input material for amplification. Inputs of 500, 50 and 5 International Units (IU) of this HIV subtype B sample (WRS: Working Reference Standard) were used. Amplification was performed as described in example 1 except that a lower KCl concentration (70mM) was used and water instead of sorbitol diluent was used to dissolve the enzymes. Results are presented in figure 3.

The results show that HIV amplification with a p1 deletion primer including up to two deletions (Figure 3D) is possible with comparable assay sensitivity as compared to the reference p1 primer (Figure 3A).

### Example 4: NASBA amplification of HIV RNA with deletion p1 and p2 primers

Part of the gag region of HIV was amplified using two different primer combinations, reference primers (HIV ref p1 (HIV.27) and HIV ref p2, table 1) and deletion primers, including both two deletions at positions where nucleotide variation occurs between different HIV subtypes (HIV deletion p1 (HIV.57) and HIV deletion p2, table 1). Amplification was performed in presence of a HIV specific molecular beacon (HIV WT MB (Me23), table 1). A dilution series of 5000, 500, 50 and 5 copies, of in vitro transcribed HIV subtype B RNA was used as input material for amplification. Amplification was performed using standard EasyQ Basic Kit NASBA reagents (BioMérieux) (40 mM Tris-HCl pH 8.5, 12 mM MgCl₂, 70 mM KCl, 15% v/v DMSO, 5 mM DTT, 1 mM each dNTP, 2 mM ATP, 2 mM CTP, 2 mM UTP, 1.5 mM GTP, 0.5 mM ITP, 0.2 µM of each primer and 0.01 µM molecular beacon probe). The mixture was incubated for 2 min at 65°C to denature the RNA and for 2 min at 41 °C, to hybridize the P1 primer to the target. Subsequently, NASBA enzymes were added, the reaction mixture was mixed by gently vortexing and short centrifugation, and the amplification and real-time detection was started. The reaction mixture was incubated at 41°C in the NucliSens EasyQ Analyzer (NucliSens, BioMérieux) for 120 minutes with fluorescence monitoring every minute. The reactions were excited at 485 nm and the emission signal was measured at 518 nm. Results are presented in figure 4. The results (figure 4B) show that HIV amplification with a deletion primer set (HIV deletion p1 (HIV.57) and HIV deletion p2, table 1) is possible and equal sensitivity is obtained as compared to the reference primer set (HIV ref p1 (HIV.27) and HIV ref p2, table 1), (figure 4A).

### Example 5: NASBA amplification of HIV RNA in presence of a deletion molecular beacon

Part of the gag region of HIV was amplified using the reference primer set (HIV ref p1 (HIV.27) and HIV ref p2, table 1)), in presence of a HIV specific molecular beacon (HIV WT MB (OTMB8472, table 1) indicated as "HIV WT reference beacon" or in presence of a HIV deletion beacon, including two deletions at positions where nucleotide variation occurs between different HIV subtypes (HIV WT deletion MB, table 1) indicated as "HIV WT deletion beacon". A dilution series of 5000, 500 and 50 copies, of in vitro transcribed HIV subtype B RNA was used as input material for amplification. Amplification was performed as described in example 1, except that the amplification was incubated for 60 minutes with fluorescence monitoring every 30 seconds. Results are presented in figure 5.

The results (figure 5B) show that HIV amplification in presence of a deletion molecular beacon is possible and equal sensitivity is obtained as compared to the reference molecular beacon (figure 5A).

### Example 6: PCR amplification of HIV DNA with deletion primers

Part of the gag region of HIV was amplified using various primer combinations, with and without deletions, in presence of a HIV specific molecular beacon (HIV WT MB (MB41, table 1)). The amplification region is the same as described in example 1 and 2, but now using PCR amplification with DNA as target and a p1 primer without T7 promoter tail. Two deletion primers were used, HIV PCR deletion p1 as reverse primer and HIV deletion p2 as forward primer, both including two deletions at positions where nucleotide variation occurs between different HIV subtypes. Four different primer combinations were tested:
1) Reference primer set, HIV PCR ref p1 with HIV ref p2,
2) HIV PCR deletion p1 with HIV ref p2,
3) HIV PCR ref p1 with HIV deletion p2 and
4) HIV PCR deletion p1 with HIV deletion p2.

A dilution series of approximately 10^6, 10^4 and 100 copies of plasmid DNA containing partly HIV subtype B sequence, was used as input material for amplification. PCR amplification was performed using a standard PCR MasterMix from Eurogentec (qPCR MasterMix No ROX) in combination with 0.3µM of both primers and 0,1µM molecular beacon. The 50µl reaction mixture was mixed by gently vortexing and short centrifugation. Subsequently the mixture was incubated for 2 min at 50 °C (UNG (Uracil-N-glycosylase) step) and 10 minutes at 95°C for UNG inactivation and HotGoldStar DNA polymerase activation, followed by 40 amplification cycles. Each cycle consists of denaturation at 95°C for 15 seconds, primer-beacon annealing at 41°C for 30 seconds plus 60°C for 20 seconds and primer extension at 72°C for 40 seconds. Fluorescence (FAM) signal is measured every cycle at the end of the 30 seconds 41°C annealing step. PCR amplification and real time detection was performed in the Mx3000p PCR cycler from Stratagene. Results are presented in figure 6. The results show that PCR amplification with a deletion primer set (HIV PCR deletion p1 and HIV deletion p2 (table 1)) is possible and equal sensitivity is obtained as compared to the reference primer set (HIV PCR ref p1 and HIV ref p2, table 1).

## Claims

1. Primer designed to amplify target nucleic acid sequence, this target sequence containing at least one variation, defined as either one non-conserved nucleotide, called genotype variation, or one nucleotide variation within one and the same genotype, wherein the primer comprises a nucleic acid sequence that is complementary to said target sequence except for the at least complementary base of the variation(s), which is not present in the primer.

2. Primer of claim 1, wherein the primer is complementary to the target sequence that contains at least two variations, each variation is separated from each other variation by at least one conserved nucleotide.

3. Primer pair to amplify target nucleic acid sequence, this target sequence containing at least one variation, defined as either one non-conserved nucleotide, called genotype variation, or one nucleotide variation within one and the same genotype, wherein at least one of the primers is designed according to claims 1 or 2.

4. Probe designed to detect target nucleic acid sequence, this target sequence containing at least one variation, defined as either one non-conserved nucleotide, called genotype variation, or one nucleotide variation within one and the same genotype, wherein the probe comprises a nucleic acid sequence that is complementary to said target sequence except for the at least complementary base of the variation(s), which is not present in the probe.

5. Probe according to claim 4, wherein it comprises at least on label.

6. Primer or primer pair or probe according to any of the claims 1-4, wherein the target sequence is a RNA target.

7. Primer or primer pair according to any of the claims 1-3, wherein one primer is provided with a promoter sequence.

8. A method of amplifying in a sample a nucleic acid target sequence containing at least one variation, defined as either one non-conserved nucleotide, called genotype variation, or one nucleotide variation within one and the same genotype, comprising performing on the sample an amplification reaction that amplifies the presence of this target sequence utilizing
1) a first primer capable of specifically hybridizing in a region of the target sequence and capable of directing, under extension conditions, extension of the first primer toward the hybridizing region of a second primer, and
2) the second primer capable of specifically hybridizing to a complementary sequence of another region of the target sequence and capable of directing, under extension conditions, extension of the second primer toward the complementary sequence of the hybridizing region of said first primer, and
wherein said at least one variation is located in the hybridizing region of at least one of the primers, and
wherein at least one of the primers comprises a nucleic acid sequence that is complementary to said target sequence except for the at least complementary base of the variation(s), which is not present.

9. A method of detecting in a sample a nucleic acid target sequence containing at least one variation, defined as either one non-conserved nucleotide, called genotype variation, or one nucleotide variation within one and the same genotype, comprising
(a) performing on the sample an amplification reaction that detects the presence of this target sequence utilizing
1) a first primer capable of specifically hybridizing in a region of the target sequence or to a complementary region of the target sequence and capable of directing, under extension conditions, extension of the first primer toward the hybridizing region of a second primer, and
2) the second primer capable of specifically hybridizing in another region of the target sequence or to another complementary region of the target sequence and capable of directing, under extension conditions, extension of the second primer toward the complementary sequence of thehybridizing region of said first primer, and
(b) performing on the sample a detection reaction that detects the presence of said target sequence, with at least one probe capable of specifically hybridizing to a region of the target sequence between the region with which the first primer is capable of hybridizing and the region with which the second primer is capable of hybridizing,
wherein said at least one variation is located in the hybridizing region of at least one of the primers, and
wherein at least one of the primers comprises a nucleic acid sequence that is complementary to said target sequence except for the at least complementary base of the variation(s), which is not present.

10. The method according to any of the claims 8 and 9, wherein the amplification reaction is selected from the group consisting of transcription-based amplification and PCR.

11. The method of claim 10, wherein the transcription-based amplification is NASBA.

12. A method according to any of the claims 8 and 9, wherein the target sequence is a RNA target.

13. A method of detecting in a sample a nucleic acid target sequence containing at least one variation, defined as either one non-conserved nucleotide, called genotype variation, or one nucleotide variation within one and the same genotype, comprising
(a) at least one probe capable of specifically hybridizing to a region of the target sequence, and
(b) detects the presence of said target sequence,
wherein said at least one variation is located in the hybridizing region of at least one of the probe(s), and
wherein at least one of the probes comprises a nucleic acid sequence that is complementary to said target sequence except for the at least complementary base of the variation(s), which is not present.

14. A method of detecting according to claim 13, wherein an amplification reaction is performed prior to the detection reaction that detects the presence of the amplicons issued from the target sequence.

15. A method of detecting according to claim 14, wherein the amplification reaction consists in
(a) adding a first primer capable of specifically hybridizing in a region of the target sequence or to a complementary region of the target sequence and capable of directing, under extension conditions, extension of the first primer toward the hybridizing region of a second primer, and
(b) adding, possibly simultaneously to the addition of the first primer, the second primer capable of specifically hybridizing in another region of the target sequence or to another complementary region of the target sequence and capable of directing, under extension conditions, extension of the second primer toward the complementary sequence of the hybridizing region of said first primer, and
(c) submitting the sample to reagents, such as enzymes, nucleotides triphosphates, and to physical conditions, such as temperature, pH, in order to permit hybridization and elongation of said primers and release of the amplicons thus obtained.

16. A method of detecting according to any of the claims 13 to 15, wherein the hybridizing region of the probe is located between the region with which the first primer is capable of hybridizing and the region with which the second primer is capable of hybridizing onto to the target sequence or on to the complementary sequence of said target sequence.

17. A method of detecting according to any of the claims 13 to 16, wherein the probe contains at least one label.

18. A method of detecting according to claim 17, wherein the probe is a nucleic acid bearing one label at each of its ends.

19. A method of detecting according to any of the claims 13 and 15 to 18, wherein the detection reaction is a real-time detection.

20. Kit containing at least one primer pair according to claim 3 and at least one probe according to any of claims 4 or 5.


SEQUENCE LISTING

<110>  bioMérieux

<120>  Method for lowering the dependency towards sequence variation of
        a nucleic acid target in a diagnostic hybridization assay.

<130>  DELPRIMER

<160>  19

<170>  PatentIn version 3.3

<210>  1
<211>  52
<212>  DNA
<213>  Hepatitis C virus

<400>  1
aattctaata cgactcacta tagggagagt tcatccacgt gcagccgaac ca          52

<210>  2
<211>  51
<212>  DNA
<213>  Hepatitis C virus

<400>  2
aattctaata cgactcacta tagggagagt tcatccacgt gcaccgaacc a          51

<210>  3
<211>  50
<212>  DNA
<213>  Hepatitis C virus

<400>  3
aattctaata cgactcacta tagggagagt tcatccacgg caccgaacca          50

<210>  4
<211>  50
<212>  DNA
<213>  Hepatitis C virus

<400>  4
aattctaata cgactcacta tagggagagt tcatccagtg caccgaacca          50

<210>  5
<211>  49
<212>  DNA
<213>  Hepatitis C virus

<400>  5
aattctaata cgactcacta tagggagagt tcatccaggc accgaacca          49

<210>  6
<211>  20
<212>  DNA
<213>  Hepatitis C virus

<400>  6
ggccgtattg ttggcattat          20

<210>  7

<211>  30
<212>  DNA
<213>  Hepatitis C virus

<400>  7
cgactgtggc ccggtatatt gcttcagtcg                                        30


<210>  8
<211>  57
<212>  DNA
<213>  Human immunodeficiency virus

<400>  8
aattctaata cgactcacta tagggagacc tgctatgtca cttccccttg gttctct          57


<210>  9
<211>  56
<212>  DNA
<213>  Human immunodeficiency virus

<400>  9
aattctaata cgactcacta tagggagacc tgctattcac ttccccttgg ttctct           56


<210>  10
<211>  56
<212>  DNA
<213>  Human immunodeficiency virus

<400>  10
aattctaata cgactcacta tagggagacc tgctatgtca cttcccctgg ttctct           56


<210>  11
<211>  55
<212>  DNA
<213>  Human immunodeficiency virus

<400>  11
aattctaata cgactcacta tagggagacc tgctattcac ttcccctggt tctct            55


<210>  12
<211>  29
<212>  DNA
<213>  Human immunodeficiency virus

<400>  12
cctgctatgt cacttcccct tggttctct                                         29


<210>  13
<211>  27
<212>  DNA
<213>  Human immunodeficiency virus

<400>  13
cctgctattc acttcccctg gttctct                                           27


<210>  14
<211>  29
<212>  DNA
<213>  Human immunodeficiency virus

<400>  14

agtgggggga catcaagcag ccatgcaaa                                    29

<210>  15
<211>  27
<212>  DNA
<213>  Human immunodeficiency virus

<400>  15
agtgggggga catcagcagc atgcaaa                                      27

<210>  16
<211>  36
<212>  DNA
<213>  Human immunodeficiency virus

<220>
<221>  misc_feature
<222>  (19)..(19)
<223>  n is inosine

<220>
<221>  misc_feature
<222>  (28)..(28)
<223>  n is inosine

<400>  16
ctatcccatc aatgaggang ctgcagantg ggatag                            36

<210>  17
<211>  36
<212>  DNA
<213>  Human immunodeficiency virus

<220>
<221>  misc_feature
<222>  (19)..(19)
<223>  n is inosine

<400>  17
ctatcccatc aatgaggang ctgcagaatg ggatag                            36

<210>  18
<211>  39
<212>  DNA
<213>  Human immunodeficiency virus

<220>
<221>  misc_feature
<222>  (19)..(19)
<223>  n is inosine

<220>
<221>  misc_feature
<222>  (28)..(28)
<223>  n is inosine

<400>  18
tgcatgcatc aatgaggang ctgcagantg ggagcatgc                         39

<210>  19

<211>  36
<212>  DNA
<213>  Human immunodeficiency virus

<400>  19
cgatgcatca atgaggagct gcagatggga gcatcg                                36